# EUROPEAN PATENT APPLICATION

(11) **EP 1 589 100 A1**
(43) Date of publication of application: **26.10.2005**
(21) Application number: 03741488.5
(22) Date of filing: 18.07.2003
(51) Int. Cl.: C12N 15/09, C07K 14/47, C07K 16/18, C12Q 1/02, C12Q 1/68, A61K 38/17, A61K 45/00, A61P 15/00, A61P 43/00

(54) **TRANSCRIPTIONAL ACTIVATOR**

(30) Priority: 28.01.2003 JP 2003018343
(71) Applicant: Japan Science and Technology Agency, Kawaguchi-shi, Saitama 332-0012 (JP)
(72) Inventor: MIYAMOTO, Kaoru, Sakai-gun, Fukui 910-0337 (JP); MIZUTANI, Tetsuya, Sakai-gun, Fukui 910-0337 (JP); KAJITANI, Takashi, Yoshida-gun, Fukui 910-1117 (JP)
(74) Representative: Wilson Gunn
(86) International application number: PCT/JP2003/009165
(87) International publication number: WO 2004/067741

(57) **Abstract**

The present invention provides a novel transcriptional factor GCX-1, which is specific to reproductive organs and may play significant roles in the hypothalamo-pituitary-gonadal axis.

The inventors isolated a novel gene, GCX-1 (SEQ ID NO: 2), which is expressed predominantly in the ovary, from a cDNA library of rat ovarian granulosa cells. Examination of abnormality in the gene or gene products (SEQ ID NO: 1) and development of medical agents effective to the transcriptional activator domain of the gene could contribute to the pathological inspection and the therapy of the diseases, such as the sterility disease, polycystic ovary syndrome, endometriosis, precocious puberty, osteoporosis and others, based on the abnormality of reproductive organs.

SEQUENCE LISTING

<110>  Japan Science and Technology Agency

<120>  TRANSCRIPTIONAL ACTIVATOR

<130>  FS03-324PCT

<160>  4

<170>  PatentIn version 3.1

<210>  1
<211>  473
<212>  PRT
<213>  Rattus norvegicus

<400>  1

Met Ser Asp Gly Asn Pro Glu Leu Leu Ser Thr Ser Gln Thr Tyr Asn
1               5                   10                  15

Ser Gln Gly Glu Ser Asn Glu Asp Tyr Glu Ile Pro Pro Ile Thr Pro
            20                  25                  30

Pro Asn Leu Pro Glu Pro Ser Leu Leu His Leu Gly Asp His Glu Ala
        35                  40                  45

Gly Tyr His Ser Leu Cys His Gly Leu Ala Pro Asn Gly Leu Leu Pro
    50                  55                  60

Ala Tyr Ser Tyr Gln Ala Met Asp Leu Pro Ala Ile Met Val Ser Asn
65                  70                  75                  80

Met Leu Ala Gln Asp Gly His Leu Leu Ser Gly Gln Leu Pro Thr Ile
                85                  90                  95

Gln Glu Met Val His Ser Glu Val Ala Ala Tyr Asp Ser Gly Arg Pro
            100                 105                 110

Gly Pro Leu Leu Gly Arg Pro Ala Met Leu Ala Ser His Met Ser Ala
        115                 120                 125

Leu Ser Gln Ser Gln Leu Ile Ser Gln Met Gly Leu Arg Ser Gly Ile
    130                 135                 140

Ala His Ser Ser Pro Ser Pro Pro Gly Ser Lys Ser Ala Thr Pro Ser
145                 150                 155                 160

Pro Ser Ser Ser Thr Gln Glu Glu Glu Ser Asp Ala His Phe Lys Ile
                165                 170                 175

Ser Gly Glu Lys Arg Pro Ser Thr Asp Pro Gly Lys Lys Ala Lys Asn
            180                 185                 190

Pro Lys Lys Lys Lys Lys Lys Asp Pro Asn Glu Pro Gln Lys Pro Val
        195                 200                 205

Ser Ala Tyr Ala Leu Phe Phe Arg Asp Thr Gln Ala Ala Ile Lys Gly
    210                 215                 220

Gln Asn Pro Ser Ala Thr Phe Gly Asp Val Ser Lys Ile Val Ala Ser
225                 230                 235                 240

Met Trp Asp Ser Leu Gly Glu Glu Gln Lys Gln Ala Tyr Lys Arg Lys
                245                 250                 255

Thr Glu Ala Ala Lys Lys Glu Tyr Leu Lys Ala Leu Ala Ala Tyr Arg
            260                 265                 270

Ala Ser Leu Val Ser Lys Ser Pro Pro Asp Gln Gly Glu Ala Lys Asn
        275                 280                 285

Ala Gln Ala Asn Pro Pro Ala Lys Met Leu Pro Pro Lys Gln Pro Met
    290                 295                 300

Tyr Ala Met Pro Gly Leu Ala Ser Phe Leu Thr Pro Ser Asp Leu Gln
305                 310                 315                 320

Ala Phe Arg Ser Ala Ala Ser Pro Ala Ser Leu Ala Arg Thr Leu Gly
                325                 330                 335

Ser Lys Ala Leu Leu Pro Gly Leu Ser Thr Ser Pro Pro Pro Pro Ser
            340                 345                 350

Phe Pro Leu Ser Pro Ser Leu His Gln Gln Leu Pro Leu Pro Pro His
        355                 360                 365

Ala Gln Gly Thr Leu Leu Ser Pro Pro Leu Ser Met Ser Pro Ala Pro
    370                 375                 380

Gln Pro Pro Val Leu Pro Ala Pro Met Ala Leu Gln Val Gln Leu Ala
385                 390                 395                 400

Met Ser Pro Ser Pro Pro Gly Pro Gln Asp Phe Pro His Ile Ser Asp
                405                 410                 415

Phe Pro Ser Gly Ser Gly Ser Arg Ser Pro Gly Pro Ser Asn Pro Ser
            420                 425                 430

Ser Ser Gly Asp Trp Asp Gly Ser Tyr Pro Ser Gly Glu Arg Gly Leu
        435                 440                 445

Gly Thr Cys Arg Leu Cys Arg Ser Ser Pro Pro Pro Thr Thr Ser Pro
    450                 455                 460

Lys Asn Leu Gln Glu Pro Ser Ala Arg
465                 470

<210>  2
<211>  1419
<212>  DNA
<213>  Rattus norvegicus

<400>  2
atgagtgacg gaaatccaga gctcctgtca accagccaga cctataacag ccagggcgag      60

agcaacgaag actatgagat ccctcctata acacctccca atctccctga gccatccctc     120

ctgcacctgg gggaccacga agccggttac cactcactgt gtcacggcct tgcgcccaac     180

ggtctgctcc ccgcctactc gtaccaggca atggatctcc cggccatcat ggtgtccaac     240

atgctggccc aggatggcca cctgctgtca ggacagctgc ccacgatcca ggaaatggtc     300

cactcggagg tagctgccta tgactcaggc cggccagggc ccctgctggg ccgccctgcg 360
atgctggcca gccacatgag tgccctcagt caatcccagc tcatctccca gatgggcctc 420
cggagtggca ttgcccacag ctccccatca cccccaggga gcaagtcagc aaccccgtct 480
ccatccagct ccacccagga ggaggagtca gatgcccatt tcaagatttc aggagagaag 540
agaccctcaa cagacccagg caaaaaggcc aaaaatccaa agaagaagaa gaagaaggat 600
cccaatgagc cacagaagcc agtgtcggcc tacgctctct tcttcagaga cactcaggct 660
gccatcaagg ggcagaaccc cagtgccacc tttggggatg tttccaaaat tgtggcctcc 720
atgtgggaca gcctgggaga agagcagaaa caggcgtata agaggaagac cgaagctgcc 780
aagaaggagt acctgaaagc cttggcggcc tacagagcta gcctcgtgtc caagagcccc 840
ccggaccaag gcgaggccaa gaacgctcag gcaaacccac cagccaaaat gcttccaccc 900
aagcagccca tgtacgccat gcccggcctg gcttccttcc tgacgccctc cgacctgcag 960
gctttccgca gcgcagcctc tcctgccagc ctcgccagaa cgctgggctc caaggccctg 1020
ctgccaggcc tcagcacctc cccaccacca ccctccttcc ctctcagccc ctccttgcac 1080
cagcagctgc cactgccacc ccacgcgcag ggtaccctcc tcagcccgcc tctcagcatg 1140
tccccagccc cgcagcctcc tgtcctgcct gcccccatgg cactccaggt gcagctggcg 1200
atgagcccct cgcctccagg gccacaggac tttccacaca tctctgactt ccccagtggc 1260
tctggctccc gctcacctgg cccatccaac ccttccagca gcggggactg ggatgggagt 1320
taccccagtg gggagcgcgg cctcggcacc tgcagactct gcagaagcag cccaccgccc 1380
accaccagcc caaagaacct gcaggaacct tctgcccgc 1419

<210> 3
<211> 21
<212> DNA
<213> Artificial sequence

<220>
<223> primer

<400> 3
cccaatgagc cacagaagcc a 21

<210> 4
<211> 20
<212> DNA
<213> Artificial sequence

<220>
<223> primer

<400> 4
ggaaagcctg caggtcggag 20

## Description

### Field of the Invention:

The present invention relates to a transcriptional activator, more specifically, to a transcriptional activator GCX-1 expressed specifically in reproductive organs.

### Prior Art

Specific events in the ovary, namely folliculogenesis, ovulation, or steroidogenesis are strictly dependent on gene expression in the related tissues. Many studies are carried out with the goal of establishing the relationships between functional events and gene expression in the ovary (Endocr Rev 15 : 725-751 (1994); Mol Cell Endocrinol 163: 61-66 (2000); Endocrinology 142: 2184-2193 (2001); Trends Endocrinol Metab 13: 1 69-173 (2002); Biol Reprod 67: 900-910 (2002).

By focusing on the molecular mechanism of follicular development, the inventors have been able to identify a number of ovarian genes induced in the rat ovary by gonadotropin treatment (Biochem Biophys Res Commun 230: 518-523 (1997); J Biol Chem 275: 22512-22519 (2000); Biol Reprod 64: 1315-1319 (2001); Biol Reprod 66: 1813-1819 (2002); Sekiguchi T et al., Transcriptional regulation of epiregulin gene in the rat ovary. Endocrinology 143: 4718-4729 (2002)). The inventors recently reported a novel transcriptional factor that is strongly induced in rat ovary by gonadotropin treatment, and designated it as GIOT-1 (gonadotropin-inducible ovarian transcription factor-1, Mol Endocrinol 15: 1693-1705 (2001)). In subsequent studies, the inventors attempted to identify proteins that interact with GIOT-1 in the ovary to reveal physiological functions of GIOT-1 and reported that a transcriptional coregulator, transcriptional intermediary factor 1 β (TIF1 β, Proc Natl Acad Sci USA 93: 1422-1426 (2001); Mol Cell Biol 18: 5880-5887 (1998); Genes Dev 15: 3023-3038 (2001)) and a transcriptional regulator, rat homologs of human I-mfa domain containing protein, (RIC, J Biol Chem 275: 4848-4857 (2000)) interact with GIOT-1 (Mol Endocrinol 15: 1693-1705 (2001)).

Transcriptional factors that are known to be expressed in gonadal tissues include Ad4BP/SF-1 and DAX-1 (Endocr Rev 18: 361-377 (1997); Recent Prog Horm Res 51: 241-260 (1996); Mol Endocrinol 10: 1261-1272 (1996)). Ad4BP/SF-1 regulates many genes that are related to steroidogenesis, including steroidogenic enzymes, StAR or GIOT-1. Ad4BP/SF-1 also plays important roles in the development of gonadal systems during embryogenesis. DAX-1 also participates in tissue development through antagonizing against the functions of Ad4BP/SF-1. However, these factors are also expressed in the adrenal gland and participate in its development and therefore are not specific to reproductive organs.

### Problems to be solved by the Invention

The present invention provides a novel transcriptional factor GCX-1, which is specific to reproductive tissues and may play significant roles in the hypothalamo-pituitary-gonadal axis, unlike Ad4BP/SF-1 and DAX-1, which are transcriptional factors expressed in reproductive tissues (Endocr Rev 18: 361-377 (1997); Recent Prog Horm Res 51: 241-260 (1996); Mol Endocrinol 10: 1261-1272 (1996)).

### Means to solve the Problems

The inventors isolated a novel clone of gene encoding a transcriptional factor-like protein containing an HMG-box domain from a cDNA library of rat ovarian granulosa cells during research on transcriptional factors in reproductive tissues (Mol Endocrinol 15: 1693-1705 (2001)). The inventors isolated this novel gene GCX-1 (granulosa cell HMG-box protein-1, SEQ ID NO: 2), determined the structure and localization of GCX-1 and analyzed the function of the gene to clarify the role of GCX-1 in the reproductive system, particularly in the ovary. Furthermore, the inventors examined the expression pattern of GCX-1 and found that GCX-1 is expressed only in tissues related to reproduction, i.e. the hypothalamus, pituitary, gonads and uterus, and also found that the GCX-1 protein is localized in the nucleus. The inventors also confirmed that the GCX-1 protein activates gene transcription, as evidenced by a GAL-4-based heterologous transcription assay.

Namely, the present invention is a transctiptional activator comprising the following (a) or (b):
(a) A protein comprising an amino acid sequence of SEQ ID NO: 1.
(b) A protein, with a transcriptional activator activity, comprising an amino acid sequence, wherein one or several amino acids are deleted, substituted or added in the amino acid sequence of (a).

The transcriptional activator activity is expressed in tissues related to reproduction, i.e. the hypothalamus, pituitary, gonads and uterus, and could be assayed by a conventional method using reporter genes.

Also, it is possible to activate intracellular transcription by introducing said factor into the cells derived from reproductive tissues for the purpose of a gene therapy.

Therefore, the present invention is a medical agent for activation of transcription, comprising the transcriptional activator of claim 1 as an effective component.

Furthermore, the present invention is an antibody or antagonist which recognizes and binds selectively to the transcriptional activator.

The antibody could be obtained from antibody producing tissues, i.e. spleen, of an appropriate animal, like a mouse, which is administrated and immunized with the transcriptional activator or its active portion according to the present invention.

Moreover, the antagonist could be obtained by screening of chemical compounds, which is able to bind to the transcriptional factor, based on the three-dimensional protein structure of the transcriptional activator or the active portion of said activator of the present invention, or by screening the peptide library, which contains peptides binding to the transcriptional activator or the active portion of said activator of the present invention using a two hybrid system.

Still furthermore, it is possible to detect abnormal production of GCX-1 by producing an antibody recognizing the amino acid sequence of GCX-1 and by examining the quantity of GCX-1 in the ovarian granulosa cells from patients (Since the cells are obtained together with ova in therapy of external fertilization, they can be applied for the examination).

Namely, the present invention is a method for examining the diseases based on the abnormality of a reproductive organ, which comprises the steps of obtaining cells from tissues of said reproductive organ of a patient and examining the reactivity of the antibody or antagonist against said cells.

In the above paragraph, a reproductive organ is referred to the hypothalamus, pituitary, gonads and uterus, and a disease based on the abnormality of a reproductive organ is referred to the sterility disease, polycystic ovary syndrome, endometriosis, precocious puberty, osteoporosis and others.

In addition, the present invention is a screening agent for medical drugs to activate the transcription, comprising the antibody or antagonist as an effective component.

Still moreover, the present invention is a transcriptional activator (GCX-1) gene comprising the following (a) or (b):
(a) DNA comprising a nucleotide sequence of SEQ ID NO: 2.
(b) DNA encoding a protein comprising an amino acid sequence of SEQ ID NO: 1 or a protein, with transcriptional activator activity, comprising an amino acid sequence, wherein one or several amino acids are deleted, substituted or added in said amino acid sequence of SEQ ID NO: 1.
   The transcriptional activator activity can be assayed by examining the expression of an appropriate reporter gene according to the method of Example 5, wherein said reporter gene is introduced into appropriate cells together with said transcriptional activator gene.
   Additionally, it is possible to inspect the abnormality of GCX-1 gene by isolating genomic DNA from the leucocytes of a patient and by examining the nucleotide sequence of said genomic DNA.
   Namely, the present invention is a method for examining the diseases based on the abnormality of a reproductive organ, which comprises the steps of obtaining DNA containing the region of the gene from a patient and comparing the obtained DNA with the nucleotide sequence of said gene.

### Brief Description of the Drawings

Figure 1 shows the deduced amino acid sequences (SEQ ID NO: 1) of GCX-1. A putative nuclear localization signal (NLS) is underlined. The putative HMG-box domain is indicated in bold type.
Figure 2 shows gene expression of GCX-1 in the rat.
Figure 3 shows *in situ* hybridization of GCX-1 in the rat ovaries. The left and right hand figures show bright-field photomicrograph and dark-field illumination, respectively. Scale bars are 0.2 mm.
Figure 4 shows identification of endogeneous GCX-1 protein.
Figure 5 shows that GCX-1 is a transcriptional activator. Each value represents the mean and SD of four independent transfection experiments.
Figure 6 shows determination of the transactivation domain of GCX-1. Each value represents the mean and SD of four independent transfection experiments.

### Description of the Invention

The inventors isolated a novel gene, GCX-1, which is expressed predominantly in the ovary, from a cDNA library of rat ovarian granulosa cells. The GCX-1 gene encodes a protein containing an HMG-box motif, a well-known DNA-binding motif that is widely distributed to all eukaryotic organisms, from yeast to mammals.

The HMG-box is one of DNA-binding motif that is widely distributed throughout eukaryotic organisms from yeast to mammals (Trends Genet 10: 94-100 (1994); Trends Biochem Sci 26: 163-174 (2001)), and a number of HMG-box proteins are known to play important roles in various physiological events: for example the upstream binding factor (UBF, Science 241: 1192-1197 (1988); Nature 344: 830-836 (1990)) as rRNA transcriptional regulator, the sex determining region Y (SRY, Nature 346: 240-244 (1990); Nature 346: 245-250 (1990)) as a protein essential for sex determination, the T cell factor (TCF, Nature 374: 70-74 (1995)) and the thymus HMG-box (TOX, Nature Imnmol 3: 272-280 (2002)) as T cell differentiation regulators. Moreover, recent studies have reported that some HMG-box proteins function not as transcriptional regulators but rather as intercellular signal transducers (Science 1999 285: 248-251 (1999); J Exp Med 1 92: 565-570 (2000); Nature 418: 191-195 (2002)). In addition, it has been reported that HMG-box proteins interact with nuclear steroid hormone receptors and function as transcriptional coregulators (Mol Cell Biol 18: 4471-4487 (1998); Steroids 64: 576-586 (1999)). Therefore, HMG-box proteins may potentially participate in the biological functions of reproductive-endocrine systems.

A protein family having an HMG-box motif is roughly divided into two groups with respect to the mode of DNA-binding. Proteins in one group, such as SRY and TCF, recognize specific DNA sequences on their target genes. Proteins belonging to the other group, such as HMGB1, UBF, and others, show nonspecific DNA-binding. The former proteins contain only one HMG-box motif, whereas the latter proteins contain two or more HMG-box motif (Mol Endocrinol 10: 1261-1272 (1996); Trends Genet 10: 94-100 (1994)).

Although GCX-1 has only one HMG-box motif, similar to specific type HMG-box proteins (Example 1), the amino acid sequence within the HMG-box of GCX-1 shows a low, but significant, similarity to those of nonspecific type HMG-box proteins (Trends Genet 10: 94- 100 (1994); Trends Biochem Sci 26: 163-174 (2001)), and has no similarity to a specific type protein. Therefore, GCX-1 may belong to a novel type of HMG-box proteins.

The gene expression of GCX-1 is limited to the ovary, testis, pituitary, and hypothalamus, suggesting that GCX-1 functions at the hypothalamo-pituitary-gonadal axis (Example 2). Transcriptional factors, Ad4BP/SF-1 and DAX-1, are essential for steroidogenesis and the embryonic development of endocrine tissues, and show gene expression patterns similar to that of GCX-1 (Endocr Rev 18: 361-377 (1997); Recent Prog Horm Res 51 : 241-260 (1996); Mol Endocrinol 10: 1261-1272 (1996)). However, these genes are also expressed in the adrenal glands, where GCX-1 is not expressed and transcriptional factors expressed only in reproductive tissues have not been known. On the other hand, gene encoding P450 aromatase (Endocr Rev 15: 342-355 (1994)) and 17 β -hydoroxysteroid dehydrogenase type I (Mol Cell Endocrinol 171: 71-76 (2001); Endocr Rev 18: 281-305 (1997)) are known to be expressed only in reproductive tissues, but they are not expressed in the pituitary gland. Therefore, GCX-1 may play an important role in the hypothalamo-pituitary-gonadal system.

An *in situ* hybridization study revealed that the strong gene expression of GCX-1 was limited to granulosa cells at the early follicle stages (Example 3), where steroidogenesis activity is still very low. Because the gene expression of GCX-1 was not observed in some other steroidogenic tissues, such as the adrenal gland or placenta, GCX-1 may play less important roles in the biosynthesis of steroid hormones. It is possible that GCX-1 may play some roles in embryonic development of reproductive tissues or in the growth and development of early-stage follicles. Events in the early-stage follicles are thought to be independent of gonadotropin stimulation. The observation that the expression of GCX-1 was not significantly changed by gonadotropin in cultured granulosa cells may be consistent with the above speculation.

Western blotting analysis of GCX-1 revealed that the GCX-1 gene product was actually present in the nucleus of ovarian granulosa cells and a GFP fusion protein analysis indicated that a putative NLS motif was essential for the nuclear translocation of GCX-1 (results not shown). In addition, a GAL4 fusion protein-based heterologous reporter assay revealed that GCX-1 functions as a strong transcriptional activator. These data suggest that GCX-1 functions in reproductive tissues as a transcriptional activator. The activation domain of GCX-1 was mapped to a region between 25 and 63 residues from N terminus. The GCX-1 also contains regions that are rich in serine, proline or lysine residues, respectively, but these regions were not essential for its transcriptional activity. Although the activation domain does not contain typical transcriptional activation motif, such as the LXXLL motif (Genes Dev 12: 3357-3368 (1998); Cell 108: 465-474 (2002); Endocrinology 143: 2461-2465 (2002)), the region is rich in hydrophobic amino acid residues, and contains LXXLL-like sequences. The LXXLL motif is known to be necessary for interactions with nuclear steroid hormone receptors or nuclear coactivators. However, the inventors were not able to confirm interactions between the GCX-1 activation domain and several known coactivators (data not shown). From the site-directed mutagenesis analyses, the inventors conclude that the entire structure of the GCX-1 activation domain might be necessary for its full activity to be exerted. The above observations suggest that the GCX-1 activation domain may represent a novel type of transactivation domain, although little information is available concerning its secondary or tertiary structure.

### Advantages of the Invention

Since the gene (SEQ ID NO: 2) and the gene product (SEQ ID NO: 1) are expressed only in reproductive system, particularly in the ovary, they could be responsible to abnormal ovulation or abnormal steroidogenesis. Therefore, examination of abnormality in the gene or gene products could contribute to the pathological inspection and the therapy of the diseases, such as the sterility disease, polycystic ovary syndrome, endometriosis, precocious puberty, osteoporosis and others, based on the abnormality of reproductive organs.

Additionally, the development of the agents effective to the transcriptional activator domain in the amino acid sequence of SEQ ID NO: 1 contributes to the medical agents for said diseases.

The following examples illustrate this invention, however, these are not constructed to limit the scope of this invention.

### Example 1

First of all, rat granulosa cells were cultured. Immature Wister rats (21 days old) were used. The rats were treated with 2 mg diethylstilbestrol (Sigma Chemical Co., St. Louis, MO) in 0.1 ml sesame oil, once daily for 4 days, to stimulate the proliferation of ovarian granulosa cells. The rats were treated always according to the guideline of National Institute of Health. The ovaries were excised, and granulosa cells were isolated by punctuating the follicles with a 26-gauge needle, and the released granulosa cells were collected. The cells were washed and collected by a brief centrifugation at 500 x g for 5 min at room temperature, and cell viability was determined by trypan blue staining. Cell viability was in excess of 90%. The granulosa cells were then cultured in Ham F-12:Dulbecco Modified Eagle Medium (1:1, vol:vol) supplemented with antibiotics and 0.1% BSA on collagen-coated plates in a humidified atmosphere containing 5% CO₂-95% air at 37°C.

In the next step, yeast two hybrid screening was conducted. A kit purchased from CLONTECH Laboratories, Inc. (Palo Alto, CA) was used for the yeast two hybrid system. All procedures were performed as described in the manufacture's instruction unless otherwise stated. The pGBKT7 vector, a parent vector for the yeast two-hybrid system, expresses the GAL4 DNA binding domain (DBD) fusion protein in yeast. The pGBKT7-GIOT1 vector, a bait plasmid, was generated as described previously (Mol Endocrinol 15: 1693-1705 (2001)). AH109 cells were transformed with the indicated bait plasmid by means of a TE/lithium acetate-based high-efficiency transformation method (Ref. Biotechniques 24: 596-600 (1998)). The construction of a plasmid cDNA library from rat granulosa cells for yeast two-hybrid screening was described (Ref. Biol Reprod 64: 1315-1319 (2001)). When a yeast strain harboring pGBKT7-GIOT-1 was transformed with the library, approximately 7 x 10⁶ primary transformants were obtained. All clones were then screened, and seven HIS3⁺/ADE2⁺/MEL1⁺ positive clones were subsequently obtained. All clones were characterized by nucleotide sequence analysis by use of a Big Dye terminator FS cycle sequencing kit and a 3100 Genetic Analyzer (Applied Biosystems Japan) and by a subsequent homology search on the Gen Bank DNA databases. A database search revealed that the nucleotide sequences of four clones showed a high similarity with the genes encoding known proteins (Mol Endocrinol 15: 1693-1705 (2001)), whereas the other three clones encoded novel proteins. From the three novel clones, a plasmid, referred to as pACT2-GCX-1, containing a 1219-bp insert with an HMG-box motif, was isolated. In a further analysis, a 758-bp EcoRI/BamHI fragment (nt-160/597) of the pACT2-GCX-1 was subcloned into the EcoRI/BamHI sites of the pBluescript II SK(+) vector (Stratagene, La Jolla, CA), and the resulting plasmid was designated as pBS-GCX-1.

Then, construction of λ -phage cDNA library and cloning of full-length GCX-1-cDNA were performed. Total RNA was prepared from granulosa cells that had been treated with 30 ng/ ml goat FSH (National Hormone and Pituitary Distribution Program, Bethesda, MD) for 3 h, by using TRIzol reagent (Invitrogen, Groningen, Netherlands). Poly(A)⁺-RNA was isolated with the oligotex dT-30 super (Roche Molecular Biochemicals, Indianapolis, IN). cDNA was synthesized from 15 *µ* g poly(A)⁺-RNA with the cDNA synthesis system and Superscript II (Invitrogen) using oligo-dT as a primer. The EcoRI/NotI adaptor was then ligated to a double-stranded cDNA, and both ends were phosphorylated with a T4 polynucleotide kinase. The cDNA was ligated to λ ZAP Express phage arms (Stratagene), followed by *in vitro* packaging using Gigapack III gold (Stratagene), to generate cDNA library. The cDNA library contained 1 x 10⁷ independent clones.

To isolate the full-length cDNA corresponding to GCX-1, the library was screened with a 758-base α-³²P dCTP -labeled EcoRI/BamHI fragment of the pBS-GCX-1, which was labeled with the BcaBest DNA labeling kit (Takara BIOMEDICALS). Eleven positive clones were isolated from approximately 60,000 cDNA clones.

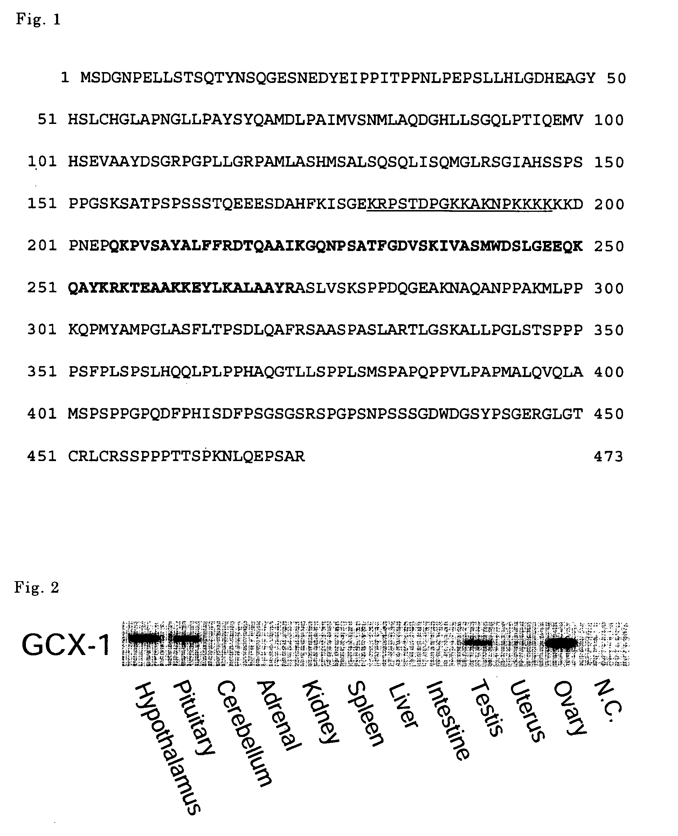

To isolate a full-length cDNA clone encoding the protein (GCX-1), the cDNA library was screened using the isolated clone as a probe, and the inventors obtained a full-length cDNA clone of the gene (SEQ ID NO: 2), which encodes a 473-amino-acid protein. The amino acid sequence (SEQ ID NO: 1) was obtained from the nucleotide sequence. As schematically drawn in Fig. 1, the protein contains one HMG-box motif (amino acids 205-272 of SEQ ID NO: 1) at the center of the protein. Therefore, the inventors refer to it as GCX-1 (granulosa cell HMG-box protein-1). It also contains a NLS (nuclear localization signal)-like motif (amino acids 180-199 of SEQ ID NO: 1) at the N-terminal side of the HMG-box, and three domains consisting of serine-rich (amino acids 124-164 of SEQ ID NO: 1), proline-rich (amino acids 348-431 of SEQ ID NO: 1), and lysine-rich (amino acids 180-199 of SEQ ID NO: 1) regions, respectively. These structural features are typical of transcriptional regulators.

### Example 2

To examine the tissue distribution of GCX-1, RT-PCR of various tissues from 21-day-old rats and Northern blot analysis were performed. Twenty-one-day-old female rats were primed with 30 IU of PMSG (Teikokuzouki, Inc., Tokyo, Japan) or with 50 IU of hCG (Sankyo Co., Ltd., Tokyo, Japan) and the ovaries were collected at the indicated times. Total RNA was extracted from various tissues (hypothalamus, pituitary, cerebellum, adrenal gland, kidney, spleen, intestine, liver, uterus and ovary) of immature female rats and from the testis of an immature male rat using TRIzol reagent. For Northern blot analysis, 10 *µ* g total RNA was separated by electrophoresis on a 1% denaturing agarose gel, transferred to a nylon membrane (Biodyne, ICN Biomedicals, Inc., Glen Cove, NY) and cross-linked by UV irradiation. For prehybridization and hybridization, ExpressHyb hybridization solution (CLONTECH) was used. A 758-base radiolabeled EcoRI /BamHI fragmant of the pBS-GCX-1, which was mentioned above, or a 1.4-kbp α -³²P dCTP-labeled BamHI fragment of the rat USF-2 (upstream stimulatory factor-2) was used as probe. Conditions for prehybridization, hybridization and washing procedures were performed according to the protocol provided by the supplier. The blot was exposed to a FUJIX imaging plate (Fuji Photo Film, Kanagawa, Japan). Hybridization signals were detected with a FUJIX BAS-2000 image analyzing system. For RT-PCR, 5 µg total RNA was reverse-transcribed and a portion (1/100) of the reaction mixture was subjected to the PCR. Primers for GCX-1 were 5'-CCCAATGAGCCACAGAAGCCA-3'(5'-primer: nt 589/609, SEQ ID NO: 3) and 5'-GGAAAGCCTGCAGGTCGGAG-3'(3'- primer: nt 936/955, SEQ ID NO: 4), respectively. Reaction conditions were 30 cycles, by denaturing at 94°C for 20 sec, annealing at 55°C for 30 sec and extending at 72°C for 45 sec using the FastStart Taq DNA Polymerase (Roche). Ten microliters of the PCR products were electrophoresed on a 1.5% agarose gel and subsequently visualized by ethidium bromide staining. The results are shown in Fig. 2.

The expression of the GCX-1 gene was detected in several restricted tissues, the hypothalamus, pituitary, testis, uterus, and ovary. The expression of GCX-1 was the highest in the ovary, whereas no expression was observed in the adrenal gland and placenta (data not shown). This strongly suggests that GCX-1 functions on the hypothalamo-pituitary-gonadal axis.

### Example 3

The expression of GCX-1 at various stages of folicular development was examined by *in situ* hybridization. For the GCX-1antisence cRNA probe, pBS-GCX-1, linearlized with EcoRI, was *in vitro* transcribed with T3 RNA polymerase (Roche) and α-³⁵S CTP. The sence probe for GCX-1 was transcribed from BamHI-digested pBS-GCX-1 with T7 RNA polymerase (Roche) and α-³⁵S CTP. Then, rat ovaries were embedded in a matrix and frozen in dry ice. Twelve to fourteen-micron-thick sections were cut by a cryostat and mounted on APS-coated glass slides for *in situ* hybridization. The sections were treated with proteinase K and were acetylated before hybridization. Hybridization with the ^{3s}S-labeled cRNA probes was performed at 60°C for 6 h and the sections were then washed under conditions of high stringency and autoradiographed using a NTB3 emulsion (Eastman Kodak Co., Rochester, NY).

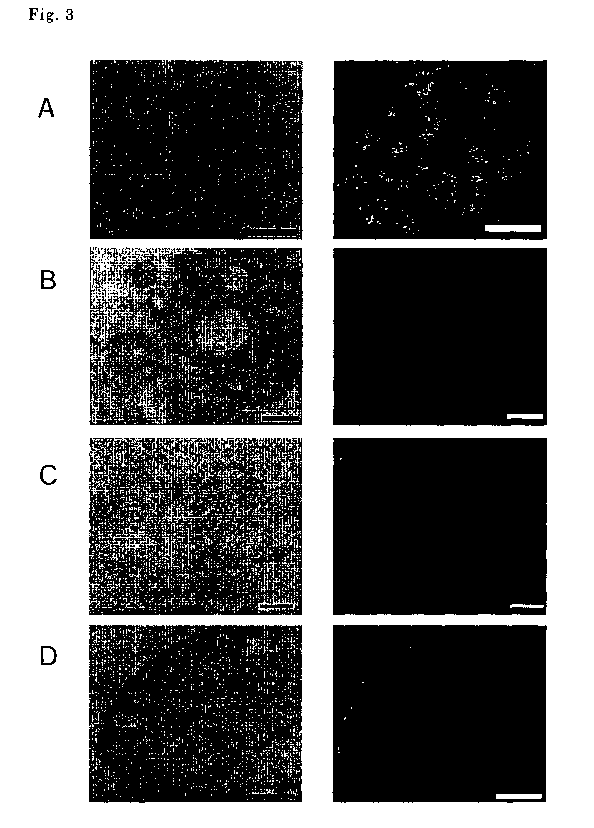

The results are shown in Fig. 3. Ovaries from 8-day-old (A), 21-day-old (B) or adult rats (C) were sectioned and hybridized with ³⁵S-labeled antisence strand cRNA probes of GCX-1. Sections of ovaries from 21-day-old rat were hybridized with sense strand cRNA probe (D).

As shown in A of Fig. 3, strong signals were detected in follicles at very early preantral stages and the expression was restricted to the granulosa cell layers. Similar levels of expression were also observed in follicles at slightly advanced stages with two or more granulosa cell layers. As shown in B of Fig. 3, signals were also detected in the follicles at large preantral and small antral stages, whereas much lower levels of expression were observed in large antral follicles at more advanced stages. Moreover, weak signals were actually detected in the corpus luteum as shown in C of Fig. 3. These observations indicate that GCX-1 gene expression is restricted to undifferentiated granulosa cells in follicles of early developmental stages.

### Example 4

An antibody against GCX-1 was prepared and endogeneous GCX-1 protein was identified. The GCX-1 specific rabbit polyclonal antiserum was generated using the peptide sequence NH₂-SLLHLGDHEAGYHSLC-CO₂H (amino acids 39-54 of SEQ ID NO: 1) and purified IgG. Granulosa cells were cultured for 24 h under hormone-free conditions in 60-mm dishes containing 5 x 10⁶ viable cells in 5 ml medium and the cells were then collected by means of a scraper and washed with 10 ml PBS. Then resulting cells were suspended in 1 ml PBS, transferred to an Eppendorf tube and pelleted by centrifugation at 1,500 x g for 10 min at 4°C. Cell extracts from granulosa cells were prepared by the method previously reported (Nucleic Acid Res 17: 6419 (1989)). The cell pellet was resuspended in 600 *µ*l cold buffer A (10mM HEPES, pH 7.9; 10 mM KCl; 1 mM EDTA; 0.5 mM EGTA; 1mM DTT; and 0.5 mM PMSF) by gentle pipetting. The cells were allowed to swell on ice for 15 min; after which, 37.5 *µ*l of a 10% solution of Nonidet P-40 was added and the tube vigorously vortexed for 10 sec. The homogenate was centrifuged at 17,000 x g for 5 min at 4°C. The supernatant was served as a cytoplasmic fraction for protein analysis and the nuclear pellet was resuspended in 40 *µ*l ice-cold buffer C (20 mM HEPES, pH 7.9; 0.4 M NaCl; 1 mM EGTA; 1mM DTT; and 1 mM PMSF) and the tube was vigorously shaken for 15 min at 4°C on a shaking-plattform. The mixture was centrifuged at 17,000 x g for 15 min at 4°C and the supernatant was recovered and used as a nuclear fraction for protein analysis. Nuclear extracts from HepG2 cells expressing GFC-GCX-fusion protein were prepared by the same protocol. The cytoplasmic and nuclear extracts (100 *µ* g each) were electrophoresed by SDS-PAGE on 10% acrylamide gel under reducing conditions. Proteins were then electrophoretically transferred to an Immobilon-P nitrocellulose membrane (Millipore Co., Bedford, MA) followed by blocking in milk buffer (58% skim milk in PBS-T[PBS containing 0.0001% of Tween-20]) and incubation with the IgG-purified GCX-1 antibody (0.019 *µ* g/ml) in milk buffer for 1 h at room temperature. The membrane was washed using PBS-T and immunoreactive GCX-1 protein was subsequently detected using the ECL-Plus kit (Amersham Biosciences Co., Piscataway, NJ) according to the manufacturer's instructions.

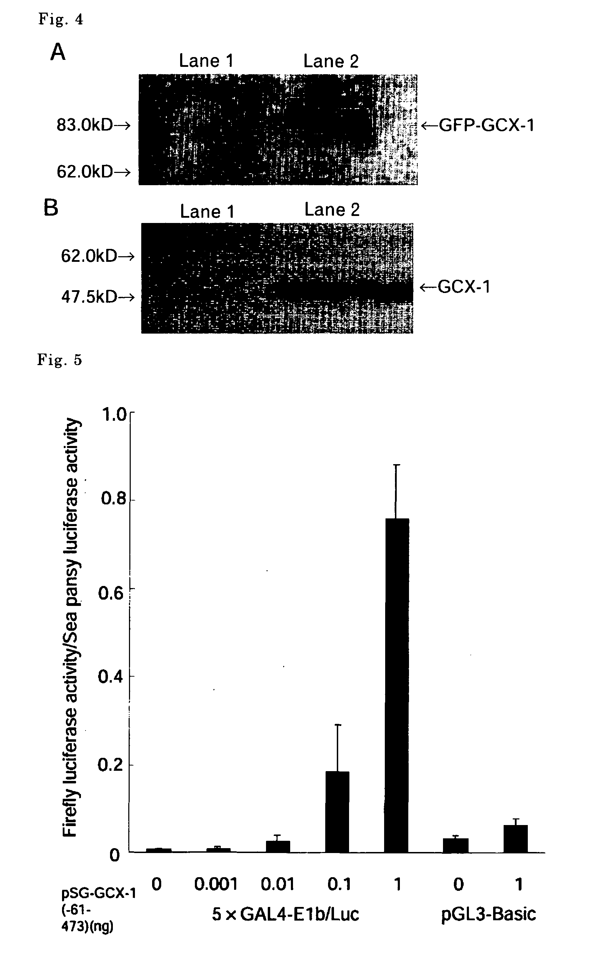

The results are shown in Fig. 4. In Fig. 4, A shows the analysis of GCX-1 specific antiserum. Nuclear extracts from pEGFP-C1E1 (lane 1)- or pEGFP-GCX-1 (-61-473) (lane 2)-transfected HepG2 cells were subjected to Western blot analysis with anti-GCX-1 antiserum (IgG purified). A specific band corresponding to the GFP-GCX-1 fusion protein is indicated. B shows the detection of endogeneous GCX-1 protein in rat ovarian granulosa cells. Cytoplasmic (lane 1) or nuclear (lane 2) extracts were separated from cultured granulosa cells and then subjected to Western blot analysis with the same antiserum. A GCX-1 specific band is indicated.

As shown in Fig. 4A, the polyclonal antibody efficiently recognized the GFP-GCX-1 protein (lane 2). The immunoreactive GCX-1 was detected only in the nuclear extracts of rat granulosa cells (Fig. 4B, lane 2) but not in the cytoplasmic fraction (Fig. 4B, lane 1).

### Example 5

Transcriptional activity of GCX-1 was investigated, since GCX-1 is a nuclear protein and contains an HMG-box motif, one of the typical motifs of transcriptional factors. A GAL4-based heterologous luciferase reporter system was used to verify the transcriptional activity of GCX-1, because target genes of this protein are not known at this time. 100 ng of the 5 x GAL4-E1b/Luc firefly luciferase reporter plasmid or the pGL3-basic reporter plasmid, and 1 ng of the pRL-CMV were cotransfected into HepG2 cells. Simultaneously, the indicated amounts of pSG-GCX-1 (-61-473) were transfected into the cells as shown in Fig. 5. pSG424, which expresses only GAL4 DBD, was added to some samples so that each sample contained the same amount of DNA. The results are shown in Fig. 5. The expression of the GAL4 DBD-GCX-1 fusion protein greatly enhanced the reporter luciferase activity. The addition of 0.1 ng of GCX-1 plasmid resulted in about a 25-fold induction of luciferase activity and 1 ng of the plasmid caused about a 100-fold induction. On the other hand, when a reporter plasmid that does not possess the GAL4 binding sequences was used instead of 5 x GAL4-E1b/Luc, no induction was observed by GCX-1 plasmid cotransfection. These observations clearly indicate that GCX-1 is a very strong transcriptional activator.

### Example 6

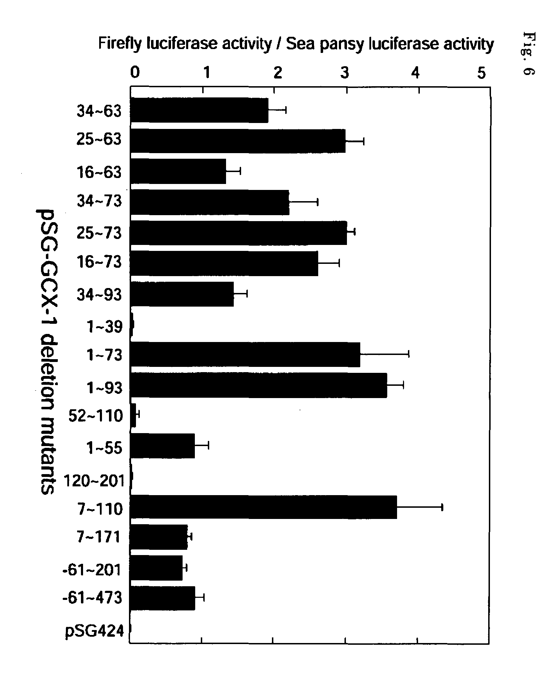

Then, the transactivation domain of GCX-1 was investigated. Various plasmid constructs of GAL4 DBD-GCX-1 deletion mutants were prepared and 1 ng of the pSG-GCX-1 deletion mutants were cotransfected with 1 ng of the pRL-CMV, 100 ng of the 5 x GAL4-E1b/Luc reporter plasmid into HepG2 cells. The results are shown in Fig. 6.

As shown in Fig. 6, a region between 25 and 63 amino acid residues from N terminus was proved to be essential for the transcriptional activity of GCX-1. The region contains no typical transcriptional activation motifs that have been reported to date.

## Claims

1. A transctiptional activator comprising the following (a) or (b):
(a) A protein comprising an amino acid sequence of SEQ ID NO: 1.
(b) A protein, with a transcriptional activator activity, comprising an amino acid sequence, wherein one or several amino acids are deleted, substituted or added in the amino acid sequence of (a).

2. A medical agent for activation of transcription, comprising the transcriptional activator of claim 1 as an effective component.

3. An antibody or antagonist which recognizes and binds selectively to the transcriptional activator of claim 1.

4. A method for examining the diseases based on the abnormality of a reproductive organ, which comprises the steps of obtaining cells from tissues of said reproductive organ of a patient and examining the reactivity of the antibody or antagonist of claim 3 against said cells.

5. A screening agent for medical drugs to activate the transcription, comprising the antibody or antagonist of claim 3 as an effective component.

6. A transcriptional activator (GCX-1) gene comprising the following (a) or (b):
(a) DNA comprising a nucleotide sequence of SEQ ID NO: 2.
(b) DNA encoding a protein comprising an amino acid sequence of SEQ ID NO: 1 or a protein, with transcriptional activator activity, comprising an amino acid sequence, wherein one or several amino acids are deleted, substituted or added in said amino acid sequence of SEQ ID NO: 1.

7. A method for examining the diseases based on the abnormality of a reproductive organ, which comprises the steps of obtaining DNA containing the region of the gene of claim 6 from a patient and comparing the obtained DNA with the nucleotide sequence of the gene of claim 6.
